# EUROPÄISCHE PATENTSCHRIFT

(11) **EP 3 902 790 B1**
(45) Veröffentlichungstag und Bekanntmachung des Hinweises auf die Patenterteilung: **03.04.2024**
(21) Anmeldenummer: 19835408.6
(22) Anmeldetag: 20.12.2019
(51) Int. Cl.: C07D 239/54

(54) **VERBESSERTES VERFAHREN ZUR HERSTELLUNG VON 4,6-DIHYDROXYPYRIMIDIN**
IMPROVED METHOD FOR THE PREPARATION OF 4,6-DIHYDROXYPYRIMIDINE
PROCÉDÉ AMÉLIORÉ DE PRODUCTION DE 4,6-DIHYDROXYPYRIMIDINE

(30) Priorität: 28.12.2018 EP 18248115
(43) Veröffentlichungstag der Anmeldung: 03.11.2021
(73) Patentinhaber: Saltigo GmbH, 51369 Leverkusen (DE)
(72) Erfinder: GROSSMANN, Andre, 50735 Köln (DE); KRAHWINKEL, Ralf, 40764 Langenfeld (DE)
(74) Vertreter: Matzke, Michael
(86) Internationale Anmeldenummer: PCT/EP2019/086766
(87) Internationale Veröffentlichungsnummer: WO 2020/136130

(56) Entgegenhaltungen:
- EP-A1- 0 816 345
- EP-A1- 1 284 261
- WO-A1-97/08152
- CN-A- 103 319 420

## Beschreibung

Gegenstand der Erfindung ist ein verbessertes Verfahren zur Herstellung von 4,6-Dihydroxypyrimidin (DHP), in seiner tautomeren Form auch 1H-Pyrimidin-4,6-dion genannt, aus Malonsäureestern, einem Ameisensäurederivat, beispielsweise Formamid, Formamidin oder Formamidiniumsalzen und Alkoholaten. DHP ist ein wertvolles Zwischenprodukt für Wirkstoffsynthesen. So kann man aus 4,6-Dihydroxypyrimidin 4,6-Dichlorpyrimidin herstellen, das seinerseits zu verschiedenen, hochwirksamen Fungiziden verarbeitet werden kann (EP-A1 0382375, EP-A1 0393861, EP-A1 0468684, EP-A1 0468695, EP-A1 2809658). Die meisten frühen Verfahren zur DHP-Herstellung basieren auf der Umsetzung von Malonsäurediamid mit Formamid in Gegenwart eines Natriumalkoholats, meistens Natriummethanolat oder Natriumethanolat, in dem entsprechenden Alkohol als Lösungsmittel (D. J. Brown in J. Chem. Soc. 1956, 2312-2314; A. Sömmer in DE-OS-1200308; V. A. Zasonov et al. in Pharmaceutical Chemistry Journal, Vol. 8, No. 12, 741-744, 1974).

Das oben beschriebenen DHP-Verfahren hat dabei zwei Eigenschaften, die als nachteilig gesehen werden können. Zum einen ist die Verwertung der Stickstoffquellen (Formamid bzw. Malonsäurediamid) schlecht, so dass mit deutlich höheren Überschüssen der Reagenzien gearbeitet werden muss und wodurch viel Ammoniak bzw. Ammoniumsalz als Abfallstrom generiert wird. Zum anderen wird eine feinkristalline Dinatrium-DHP-Zwischenstufe bei der Kristallisation durchlaufen, weshalb die Filtration des Produktes gegebenenfalls sehr langsam verläuft und Taktzeit des Prozesses sehr lang wird.

Ein verbessertes Verfahren beschreiben dagegen die beiden Patente von Degussa AG (EP-A-0 816 345 und EP-A-1 284 261). So ist in EP-A-1 284 261 das Verfahren zu DHP so dargelegt, dass nur noch 2,25 mol Formamid auf 1 mol Malonsäuredimethylester benötigt wird. Durch die Verwendung von Natriummethanolat als Base in einem Autoklaven unter Druck entsteht so das DHP in Ausbeuten von 84 bis 91 Prozent der Theorie.

Formamidin und Formamidiniumsalze (Acetat oder Hydrochlorid) sind ebenfalls als Stickstoffquellen statt Formamid zur Synthese von DHP in ähnlichen Verfahren bereits bekannt (CN 103 319 420).

Nach dem aktuellen Stand der Technik haben alle soweit bekannten Verfahren zur Herstellung des DHP zwei weitere Nachteile, die bisher nicht behoben waren. Zur Herstellung eines Pyrimidin-Derivates aus Formamid und einem Malonsäureester werden stets aus chemisch fundamentalen Gründen mindestens drei Äquivalente Alkoholat, bezogen auf Malonsäureester gerechnet, benötigt. Nach der Reaktion zum Pyrimidin entstehen aus diesen Basen die entsprechenden Alkohole, die mit diversen Aminverbindungen aus Nebenreaktionen und wässrigen Salzlösung verunreinigt sind. Zum Beispiel fallen pro kg DHP laut Beispiel 3 des Patentes EP-A-0 816 345 3,83 kg Methanol an, das aufwendig aus der Mutterlauge destillativ wiedergewonnen und in anderen Verfahren etwaige Verwendung finden muss. Alternativ muss dieses verunreinigte Methanol entsorgt werden, beispielsweise durch Verbrennung. Eine direkte Verwendung des Alkohols zur Herstellung von DHP ist dagegen kaum möglich, da die Herstellung von Methanolaten, Ethanolaten und Propylaten nur über Elektrolyse von Alkalihalogeniden in Methanol, Ethanol bzw. Propanol erfolgen kann und für diesen elektrochemischen Vorgang eine dedizierte Anlage benötigt wird. Alternativ können Alkoholate durch die Reaktion des Alkohols mit metallischem Natrium hergestellt werden, wobei jedoch Wasserstoff erzeugt wird und daher in einem industriellen Herstellungsprozess sicherheitstechnische Aspekte dagegen sprechen. Die aktuellen Verfahren zu DHP haben somit klare wirtschaftliche und umwelttechnische Nachteile.

Es bestand daher die Aufgabe, ein Verfahren zur Herstellung von 4,6-Dihydroxypyrimidin bereitzustellen, dass zum einen das Produkt in hoher Ausbeute liefert und vor allem diese beiden Nachteile nicht aufweist.

Es wurde nun ein weiterhin verbessertes Verfahren zur Herstellung von 4,6-Dihydroxypyrimidin gefunden, umfassend zumindest die Schritte
a) Bereitstellung eines Alkalimetallalkoholats der Formel (I),

   R¹-OM (I)

   in der R¹ für n-Butyl, Isobutyl und sec-Butyl und M für Natrium und Kalium steht,
b) Umsetzung von Malonsäureester der Formel (II), 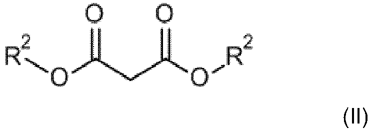
   in der R² für C₁ bis C₄ Alkyl steht,
   mit einem Ameisensäurederivat der Formel (III), 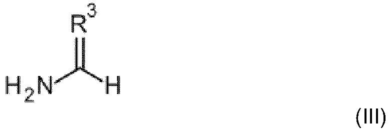
   in der der Rest R³ für O, HN oder NH⁺X⁻ steht, wobei X⁻ für das Anion einer Säure, bevorzugt für Chlorid oder Acetat steht,
   in Gegenwart des Alkalimetallalkoholats der Formel (I),
   wobei das Alkalimetallalkoholat der Formel (I) in Schritt a) durch Umsetzung von zumindest einem Alkalihydroxid der Formel (IV),

   M-OH (IV),

   in der M die in Formel (I) angegebene Bedeutung hat,
   mit einem Alkohol der Formel (V),

      R¹-OH (V),
   in der R¹ die in Formel (I) genannte Bedeutung hat, entweder in Reinsubstanz oder in Form einer Mischung, unter Abdestillieren von Wasser und Alkohol der Formel (V), bis der Destillationsrückstand einen Gehalt an Alkalihydroxid der Formel (IV) von maximal 1000 mg/kg, bevorzugt von maximal 300 mg/kg, bezogen auf das Gesamtgewicht der Mischung, aufweist, bereitgestellt wird.

In Schritt a) wird ein Alkalimetallalkoholat der Formel (I) bereitgestellt, in der Rest R¹ für n-Butyl und der Rest M für Natrium oder Kalium steht.

In Schritt b) wird ein Malonsäureester der Formel (II) verwendet, in dem der Rest R² für Methyl, Ethyl oder n-Butyl steht.

Besonders bevorzugt ist das Ameisensäurederivat der Formel (III) Formamid (Rest R³ steht für O), Formamidin (Rest R³ steht für N), Formamidiniumsalze (Rest R³ steht für NH⁺X⁻), wobei der Rest X⁻für das Anion einer Säure, beispielsweise im Fall von Formamidiniumchlorid für Chlorid oder im Fall von Formamidiniumacetat für Acetat steht.

In einem Schritt c) erfolgt üblicherweise die Reaktion der bei Schritt b) resultierenden Mischung bei einer Temperatur von 50 bis 110°C, bevorzugt von 60 bis 80°C. Bevorzugt erfolgt Schritt c) nach Schritt b) und oder gleichzeitig zu Schritt b).

Während der Schritte b) und c) erfolgt die Kondensationsreaktion zwischen Malonsäureester und Ameisensäurederivat zu einem 4,6-Dihydoxypyrimidiniumsalz (DHP-Salz), das pro Molekül DHP-Salz zwei Kationen M⁺ aufweist.

Das erfindungsgemäße Verfahren weist eine Reihe von überraschenden Vorteilen gegenüber den Verfahren des Standes der Technik auf. Der bei der Reaktion entstehende Alkohol lässt sich im Gegensatz zu Methanol und Ethanol leicht aus dem zweiphasigen Filtrat, das nach der Abtrennung des DHP aus dem Reaktionsgemisch erhalten wird und das neben Alkohol, Wasser und Salze enthält, durch einfache Phasentrennung wiedergewinnen. Eine aufwändige destillative Aufarbeitung eines Filtrats, das Alkohol und Wasser in einer Phase enthält, nach der Isolierung des gewünschten Produkts DHP ist nicht mehr erforderlich.

Weiterhin und ebenfalls in Gegensatz zu den Verfahren gemäß dem Stand der Technik kann aus dem Alkohol der Formel (V) und Natronlauge durch einfache azeotrope Destillation die Natriumalkoholat-Base leicht wiedergewonnen werden. Unter dem Strich vereinfacht sich also die Massenbilanz der DHP-Synthese dahingehend, dass als Base letztendlich Natronlauge dient und fast kein Abfall in Form von etwaigen organischen Verbindungen, beispielsweise Alkohole, mehr anfällt.

Die Reaktion von Malonsäureester der Formel (II) mit Ameisensäurederivat der Formel (III) ist nachfolgend schematisch dargestellt. Die Reaktion erfolgt in Gegenwart von Alkalimetallalkoholat der Formel (I) mit nachfolgender Freisetzung des DHP durch Säure.

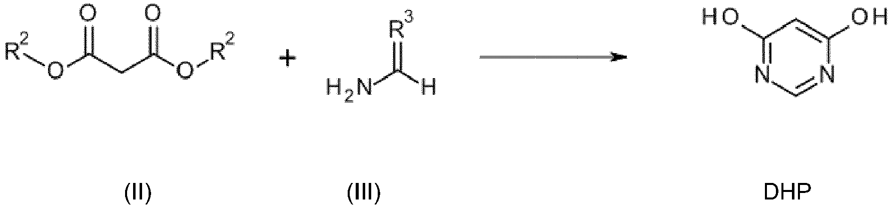

In dem Reaktionsschema steht der Rest R³ in dem Ameisensäurederivat der Formel (III) für O im Fall von Formamid und für HN im Fall von Formamidin und für NH⁺X⁻ im Fall von Formamidiniumsalzen. Der Rest X steht dabei für das Anion einer Säure, beispielsweise für Chlorid bei Formamidiniumhydrochlorid oder Acetat bei Formamidiniumacetat.

Ein zusätzlicher Vorteil des erfindungsgemäßen Verfahrens ist, dass dieses Reaktionsgemisch bei den benötigten Reaktionstemperaturen keinen Druck aufbaut und somit der apparativer Aufwand des Verfahrens vereinfacht wird.

Weiterhin ist es im erfindungsgemäßen Verfahren möglich das Produkt so zu kristallisieren, dass das nicht hydrolysierte Reaktionsgemisch parallel mit einer wässrigen Salzsäure auf Wasser dosiert werden kann. Überraschenderweise kann hierdurch im neuen Verfahren die bisher höchste in der Literatur beschriebene Ausbeute von DHP von 92,5% der Theorie und mit einem Gehalt von >98 Gew.% (HPLC-ESTD Methode) die bisher höchste in der Literatur beschriebene Qualität erreicht werden.

Im Folgenden wird das erfindungsgemäße Verfahren detaillierter beschrieben:
Als Alkalimetallalkoholat der Formel (I) werden Natrium- und Kalium-Alkoholate, insbesondere Natriumalkoholate verwendet. Der Rest R¹ im Alkalimetallalkoholat der Formel (I) R¹-OM steht für n-Butyl-, sec-Butyl und iso-Butyl. Alkohole mit weniger Kohlenstoffatomen sind für dieses Verfahren ebenso wenig geeignet wie Alkohole mit mehr als vier Kohlenstoffatomen. Alkalimetallalkoholate der Formel (I) basierend auf tert.-Butanol sind für das erfindungsgemäße Verfahren ebenfalls nicht geeignet. Besonders bevorzugt ist Natrium-n-butanolat.

In dem erfindungsgemäßen Verfahren zur Herstellung von 4,6-Dihydroxypyrimidin wird das Alkalimetallalkoholat der Formel (I) in Schritt a) durch Umsetzung von zumindest einem Alkalihydroxid der Formel (IV),

M-OH (IV),

in der M die in Formel (I) angegebene Bedeutung hat, mit einem Alkohol der Formel (V),

R¹-OH (V),

in der R¹ die in Formel (I) genannte Bedeutung hat, entweder in Reinsubstanz oder in Form einer Mischung, unter Abdestillieren von Wasser und Alkohol der Formel (V), bis der Destillationsrückstand einen Gehalt an Alkalihydroxid der Formel (IV) von maximal 1000 mg/kg, bevorzugt von maximal 300 mg/kg, bezogen auf das Gesamtgewicht der Mischung, aufweist, bereitgestellt.

Das Alkalimetallalkoholat der Formel (I) kann dabei aus einem Gemisch des entsprechenden Alkohols R¹-OH und Alkalihydroxids M-OH hergestellt werden. Dies gelingt beispielsweise sowohl durch eine Azeotropierung, beispielsweise in einem Batchreaktor, wie auch durch eine kontinuierliche Destillation, beispielsweise in einem Rohrreaktor. Für das Verfahren zur Herstellung von DHP ist es wichtig, dass das gebildete Alkalimetallalkoholat maximal einen Restgehalt an Alkalihydroxid von 1000 mg/kg, bevorzugt von maximal 300 mg/kg, erhält, da sich höhere Gehalte negativ auf die Qualität von DHP auswirken. Das Alkalimetallalkoholat der Formel (I) kann direkt nach seiner Herstellung in Schritt a) bereitgestellt oder auch zeitlich vorher hergestellt und dann gelagert werden. Allerdings ist zu beachten, dass beispielsweise eine Lösung von Natrium-n-butanolat in Butanol mit einem Gehalt an Natrium-n-butanolat von mindestens 25 Gew.% bei Temperaturen unter 60°C zu einer Schmelze erstarrt und gegenüber Luftsauerstoff nicht stabil ist. Für die Umsetzung zu DHP wird das Alkalimetallalkoholat der Formel (I) deshalb vorzugsweise entweder unter Sauerstoffausschluss, beispielsweise unter Schutzgas, aufbewahrt oder direkt weiter zu DHP umgesetzt.

Die Qualität des Alkalimetallalkoholats der Formel (I) in Bezug auf den Restgehalt an Natriumhydroxid wird indirekt mittels Wasserbestimmung nach Karl-Fischer verifiziert. Hierzu wird eine entnommene Probe des Alkalimetallalkoholats der Formel (I), beispielsweise von Natrium-n-butanolat in Form seiner Schmelze, in wasserfreier, organischer Säure, bevorzugt Essigsäure, zunächst aufgelöst, wobei das entsprechende Natriumsalz der Säure, beispielsweise Natriumactetat, und Wasser entstehen, und das Wasser über die Karl-Fischer-Methode bestimmt wird.

In dem Malonsäureester der Formel (II) steht der Rest R² für C₁ bis C₄-Alkyl, bevorzugt für Methyl oder Ethyl.

Alle Reagenzien und Edukte können sowohl in Form hochreiner als auch technischer Produkte verwendet werden.

Im erfindungsgemäßen Verfahren erfolgt die Umsetzung von Malonsäureester der Formel (II) mit dem Ameisensäurederivat der Formel (III) in Gegenwart des Alkalimetallalkoholats der Formel (I) in Schritt b) bei einer Temperatur von 50 bis 110°C, bevorzugt von 60 bis 80°C.

In diesem Schritt b) liegt üblicherweise das Alkalimetallalkoholat der Formel (I) in Form einer Suspension, einer Schmelze oder als Lösung, vorzugsweise mit dem korrespondierenden Alkohol der Formel (V) als Lösungsmittel, vor.

Bevorzugt erfolgt die Umsetzung von Malonsäureester der Formel (II) und Ameisensäurederivat der Formel (III) in Gegenwart des Alkalimetallalkoholats der Formel (I) derart, dass Alkalimetallalkoholat der Formel (I) vorgelegt und Malonsäureester der Formel (II) und Ameisensäurederivat der Formel (III) zu dem Alkalimetallalkoholat der Formel (I) zugegeben werden.

Üblicherweise kann das Ameisensäurederivat der Formel (III) entweder allein oder gleichzeitig mit der gesamten oder einer Teilmenge des Malonsäureester der Formel (II), portionsweise oder kontinuierlich dem Alkalimetallalkoholat der Formel (I) und gegebenenfalls der gesamten oder der restlichen Teilmenge des Ameisensäurederivats der Formel (III) zugefügt werden. Es ist dabei vorteilhaft Malonsäureester der Formel (II) und Ameisensäurederivat der Formel (III) als Gemisch kontinuierlich zu dosieren. Dabei ist im erfindungsgemäßen Verfahren bevorzugt, dass in Schritt b) die Umsetzung von Malonsäureester der Formel (II) und Ameisensäurederivat der Formel (III) in Gegenwart des Alkalimetallalkoholats der Formel (I) derart erfolgt, dass während eines beliebigen Zeitpunktes der Umsetzung das molare Verhältnis der Summe des bis zu diesem Zeitpunkt zugegebenen Ameisensäurederivats der Formel (III) zu der Summe des bis zu diesem Zeitpunkt zugegebenen Malonsäureesters der Formel (II) mindestens 2,05, bevorzugt mindestens 2,5 bis 3,5, beträgt.

Dieses molare Verhältnis wird beispielsweise in einer Ausführungsform dadurch erreicht, dass die gesamte Menge an Ameisensäurederivat der Formel (III) zu dem Alkalimetallalkoholat der Formel (I) zugegeben wird, bevor der Malonsäureester der Formel (II) dieser daraus entstandenen Mischung zugegeben wird. Werden beispielsweise 2,05 Mol, oder bevorzugt 2,5 Mol, Ameisensäurederivat der Formel (III) dem Alkalimetallalkoholat der Formel (I) zugegeben, bevor irgendeine Menge an Malonsäureester der Formel (II) zugegeben wurde, dürfen anschließend maximal 1,0 mol Malonsäureester der Formel (II) zugegeben werden, damit am Ende der Zugabe, also an einem beliebigen Zeitpunkt der Zugabe, das molare Verhältnis der Summe des bis zu diesem Zeitpunkt zugegebenen Ameisensäurederivat der Formel (III) zu der Summe des bis zu diesem Zeitpunkt zugegebenen Malonsäureesters der Formel (II) mindestens 2,05, oder bevorzugt 2,5, beträgt. Während der Zugabe von Malonsäureester der Formel (II) ist das molare Verhältnis zu jedem früheren Zeitpunkt größer als 2,05, oder bevorzugt 2,5. In einer anderen Ausführungsform können beispielsweise zunächst 0,1 Mol Ameisensäurederivat der Formel (III) dem Alkalimetallalkoholat der Formel (I) zugegeben werden, bevor irgendeine Menge an Malonsäureester der Formel (II) zugegeben wurde. Danach können beispielsweise Ameisensäurederivat der Formel (III) und Malonsäureester der Formel (II) der entstandenen Mischung im molaren Verhältnis von 2,05, oder bevorzugt von 2,5, simultan zudosiert werden. Auch dann beträgt zu einem beliebigen Zeitpunktes der Zugabe das molare Verhältnis der Summe des bis zu diesem Zeitpunkt zugegebenen Ameisensäurederivats der Formel (III), zu der Summe des bis zu diesem Zeitpunkt zugegebenen Malonsäureesters der Formel (II) mindestens 2,05, bevorzugt mindestens 2,5 bis 3,5. Weitere Ausführungsformen können in der diskontinuierlichen oder kontinuierlichen Zugabe der beiden Reaktanden zu dem Alkalimetallalkoholat der Formel (I) bestehen, solange das oben definierte molare Verhältnis zu jedem Zeitpunkt der Zugabe zumindest eines der Reaktanden eingehalten wird. Erfindungsgemäß wird dabei kontinuierliche Zugabe derart definiert, dass sie ohne Unterbrechung erfolgt. Diskontinuierliche Zugabe bedeutet erfindungsgemäß, dass die Zugabe, beispielsweise in mehreren diskreten Portionen, mit Unterbrechungen erfolgt. Dabei können bei der diskontinuierlichen Zugabe sowohl zeitliche Phasen kontinuierlicher und ebenso zeitliche Phasen mit diskontinuierlicher Zugabe enthalten sein.

Die Temperatur im Reaktionsgemisch, das in Schritt b) vorliegt, wird zweckmäßig im Bereich von 50 bis 110 °C, insbesondere von 60 bis 80 °C gehalten. Die Reaktion ist schwach exotherm, so dass gegebenenfalls gekühlt werden muss, sobald mit der Zugabe von Malonsäureester der Formel (II) begonnen wurde. Je nach den eingesetzten Stoffmengen kann die Zugabe des Malonsäureester der Formel (II) und gegebenenfalls des Ameisensäurederivats der Formel (III) im allgemeinen etwa von 10 bis 120 Minuten, bevorzugt von 20 bis 30 Minuten in Anspruch nehmen. Längere Dosierzeiten sind ebenfalls vorstellbar, bringen aber neben Zeitverlust keinen Vorteil für das Verfahren mit sich. Weiterhin vorteilhaft wird das Reaktionsgemisch in Schritt b) nach beendeter Zugabe einige Zeit, beispielsweise von 20 bis 60 Minuten, bevorzugt von 30 bis 40 Minuten, bei einer Temperatur von 50 bis 110°C, bevorzugt von 60 bis 80°C, weiter vermischt. Das Vermischen kann beispielsweise mechanisch, vorzugsweise mit einem Rührer, oder hydraulisch, vorzugsweise durch Umpumpen, erfolgen.

Das abreagierte Reaktionsgemisch aus Schritt c), das das Produkt DHP in der Form des Dialkalimetallsalz mit M⁺ als Kation enthält, wird in das Produkt DHP durch Inkontaktbringen mit Säure überführt. Vorzugsweise erfolgt in Schritt
d) das Inkontaktbringen von anorganischer Säure und Wasser mit der Reaktionsmischung aus Schritt c), die ausreicht, um die Mischung auf einen pH-Wert von 2 bis 5, bevorzugt von pH 3 bis 4, zu bringen.

Das Inkontaktbringen kann dabei beispielsweise durch Vermischen erfolgen, wobei das Vermischen vorzugsweise mechanisch, besonders bevorzugt mit einem Rührer, oder hydraulisch, besonders bevorzugt durch Umpumpen, erfolgen. Das Inkontaktbringen von anorganischer Säure und Wasser zu der Mischung aus Schritt c) in Schritt d) kann dabei diskontinuierlich oder kontinuierlich erfolgen. Durch das Vermischen von anorganischer Säure und Wasser mit der Reaktionsmischung aus Schritt c) wird eine homogene Einstellung des pH-Wertes innerhalb des daraus entstehenden Reaktionsgemisches erreicht. Die dadurch erreichte Hydrolyse des Dialkalimetallsalzes vom DHP kann in demselben Reaktor durchgeführt werden, in dem zuvor die Kondensationsreaktion erfolgte. Hierbei kann zunächst Wasser zu dem Reaktionsgemisch gegeben und dann mit wässriger Säure der pH auf 3 bis 4 eingestellt werden. Diese Vorgehensweise hat aber gegebenenfalls negative Auswirkungen auf die Qualität und Filtrierbarkeit des Produktes. In einer bevorzugten Ausführungsform von Schritt d) wird Wasser vorgelegt, dann zunächst das Reaktionsmischung aus Schritt c) und nachfolgend Säure, gegebenenfalls gelöst in Wasser, zugegeben, bis der erforderliche pH-Wert der daraus resultierenden Mischung erreicht wurde. In einer weiteren bevorzugten Ausführungsform von Schritt d) wird das Reaktionsmischung aus Schritt c) vorgelegt, dann zunächst Wasser und nachfolgend Säure, gegebenenfalls gelöst in Wasser, zugegeben, bis der erforderliche pH-Wert der daraus resultierenden Mischung erreicht wurde. In einer besonders bevorzugten Ausführungsform von Schritt d) wird Wasser vorgelegt und dann parallel unter Vermischung die Reaktionsmischung aus Schritt c) und Säure, gegebenenfalls gelöst in Wasser, derart zugegeben, dass dabei der pH bei 2 bis 5, bevorzugt bei pH 3 bis 4, gehalten wird. Als Säure wird üblicherweise ein anorganische Säure, bevorzugt Chlorwasserstoff, besonders bevorzugt wässrige Salzsäure, in Schritt c) eingesetzt.

Das in Schritt d) erhaltenen Reaktionsgemisch enthält als Feststoff ausgefälltes DHP, das in zwei flüssigen Phasen suspendiert ist. In Schritt e) des erfindungsgemäßen Verfahrens erfolgt die Trennung des aus Schritt d) erhaltenen Reaktionsgemisches, wobei 4,6-Dihydroxypyrimidin als Feststoff und ein zweiphasiges Filtrat erhalten wird, wobei eine Phase zumindest überwiegend den Alkohol der Formel (V) enthält. Die zweite flüssige Phase enthält überwiegend Wasser.

Diese Trennung erfolgt üblicherweise durch Filtration oder Zentrifugation. Das isolierte DHP wird üblicherweise mit Wasser gewaschen und zweckmäßig bei erhöhter Temperatur, beispielsweise von 50 bis 90°C, und unter vermindertem Druck, beispielsweise von 2 bis 20 KPa, getrocknet. Man erhält mit dem erfindungsgemäßen Verfahren das Reaktionsprodukt in einer Reinheit von mindestens 98 Gew.% mit Ausbeuten, die üblicherweise 90% der Theorie überschreiten.

Das erfindungsgemäße Verfahren hat neben der hohen erzielten Ausbeute und hohem chemischen Reinheit des darüber hergestellten DHP den wesentlichen Vorteil, dass das in Schritt e) nach der Abtrennung des 4,6-Dihydroxypyrimidins verbleibende zweiphasige Filtrat in eine wässrige und eine organische Phase, die zumindest überwiegend den Alkohol der Formel (V) enthält, aufgetrennt werden kann. Danach wird bevorzugt die organische Phase einmal ohne Fraktionierung destilliert, wobei als Rückstand Salze und andere feste organische Verunreinigungen zurückbleiben. Das Destillat weist dann üblicherweise einen Anteil an Alkohol der Formel (V) von 80 bis 99 Gew.-% auf, was mittels Gaschromatographie überprüft werden kann. Das Destillat kann dann bevorzugt als Alkohol der Formel (V) zur Herstellung des Alkalimetallalkoholats der Formel (I) verwendet werden, das anschließend in einen weiteren Schritt b) zur Herstellung von DHP nach dem erfindungsgemäßen Verfahren eingesetzt werden kann. Dadurch wird ein wesentlich effizienteres Verfahren erreicht, in dem die bei der Abtrennung des Reaktionsprodukts entstehende organische flüssige Phase überwiegend wiedergewonnen und in eine nachfolgende Reaktion des gleichen Typs eingesetzt werden kann. Das Produkt DHP wird aufgrund seiner der vielfältigen Anwendungen im Kilotonnenmaßstab benötigt. Durch das erfindungsgemäße Verfahren wird dadurch eine wesentliche Menge an Abfallstoffen reduziert, die ansonsten kostenintensiv und unter CO₂-Emissionen, beispielsweise durch Verbrennung, entsorgt werden müssen. Die Qualität des mit dem erfindungsgemäßen Verfahren hergestellten Produkt DHP wird überraschenderweise durch diese Rezyklierung nicht nachteilig beeinflusst.

Ein weiterer Gegenstand der Erfindung ist somit die Verwendung der Alkohole der Formel (V) und/oder ihrer Alkalimetallalkoholate der Formel (I) zur Herstellung von 4,6-Dihydroxypyrimidin.

### Beispiele

### Beispiel 1 - Natrium-n-butanolat Herstellung (erfindungsgemäß)

In einem 1-L Planschlifftopf, der mit einer Kolonne mit mindestens 10 theoretischen Böden und oberhalb der Kolonne mit einem Wasserabscheider versehen wurde, wurden 80 g 50%ige Natronlauge (1,00 mol) und 800 g n-Butanol (10,79 mol) vorgelegt. Das Gemisch wurde zum Siedepunkt (ca. 90 °C) bei 200 mbar erhitzt und solange azeotropiert, bis sich kein Wasser mehr abschied. Anschließend wurde das Gemisch zusätzlich destillativ so aufkonzentriert, bis die Temperatur im Sumpf bei 200 mbar von 104 bis 105°C erreicht wurde. Die gebildete Natrium-n-butanolat-Schmelze erstarrt bei unter 60 °C und ist gegenüber Luftsauerstoff nicht stabil. Für die Umsetzung zu DHP wurde sie entweder unter strickten Sauerstoffausschluss aufbewahrt oder direkt weiter zu DHP umgesetzt. Das so hergestellte Natrium-n-butanolat wies einen Natrium-n-butanolat-Gehalt von 33 Gew.-% und Natriumhydroxid-Restgehalt von unter 1000 mg/kg auf.

### Beispiel 2 - Natrium-n-butanolat Herstellung (erfindungsgemäß)

Beispiel 1 wurde mit einer organischen flüssigen Phase aus einer vorherigem Herstellung von DHP als Ersatz für reines n-Butanol wiederholt, wobei die organische Phase nach Abtrennung der wässrigen Phase nach einer Destillation ohne Fraktionierung erhalten wurde. Die organische flüssigen Phase wies einen Gehalt von n-Butanol von 85,6 Gew.-% auf. Das so hergestellte Natrium-n-butanolat-Schmelze wies einen Natrium-n-butanolat-Gehalt von 33 Gew.-% und Natriumhydroxid-Restgehalt von unter 1000 mg/kg auf. Die Fremdbestandteile im recycelten n-Butanol, hauptsächlich Methanol aus dem Malonsäuredimethylester, wurden mit dem abgeschiedenen Wasser bei der azeotropen Destillation über Kopf der Kolonne entfernt.

### Beispiel 3 - Herstellung von 4,6-Dihydroxypyrimidin mit Formamid (erfindungsgemäß)

In einem 1-L Planschlifftopf wurden 450 g Natrium-n-butanolat (33 Gew.-% Gehalt; 1,55 mol) in Butanol vorgelegt und auf 70 °C temperiert. Anschließend wurden 1,18 Mol Formamid und 0,40 Mol Dimethylmalonat als Gemisch über 60 min zudosiert. Nach Abschluss der Dosierung wurde das Reaktionsgemisch 30 min nachgerührt und auf 30 °C abgekühlt.

In zweiten 1-L Planschlifftopf wurden 250 g entsalztes Wasser vorgelegt. Die Reaktionssuspension aus dem ersten Reaktor wurde dann parallel mit wässriger Salzsäure (30 Gew.-%ig) in den zweiten Reaktor so zudosiert, dass der pH bei 3-4 lag und die Temperatur unter 30 °C blieb. Die wässrige Produktsuspension wurde 60 min bei unter 30 °C nachgerührt und abgesaugt. Das Produkt wurde drei Mal mit jeweils 50 g entsalzten Wasser nachgewaschen und bei 70 °C und ca. 100 mbar getrocknet. Die Ausbeute an DHP betrug 41,7 g, entsprechend 92,5% der Theorie. Der Gehalt lag bei 99,5 Gew.-% (absolute Gehaltsbestimmung über HPLC mit externem Standard).

Die Mutterlauge aus der DHP-Filtration wurde phasengetrennt und die organische Phase wurde einmal ohne Kolonne thermisch überführt, um die Salze und feste organischen Verunreinigungen abzutrennen. Das so wiedergewonnene Butanol wurde analog zu Beispiel 1 anschließend erneut zur Herstellung von Natrium-n-butanolats ohne Nachteile gegenüber einem kommerziell erhältlichen n-Butanol verwendet werden (siehe Beispiel 2).

## Patentansprüche

1. Verfahren zur Herstellung von 4,6-Dihydroxypyrimidin umfassend zumindest die Schritte
a) Bereitstellung eines Alkalimetallalkoholats der Formel (I),
R¹-OM (I)
in der R¹ für n-Butyl, Isobutyl und sec-Butyl und M für Natrium und Kalium steht,
b) Umsetzung von Malonsäureester der Formel (II), 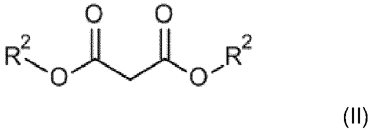
in der R² für C₁ bis C₄-Alkyl steht,
mit einem Ameisensäurederivat der Formel (III), 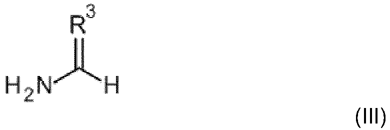
in der der Rest R³ für O, HN oder NH⁺X⁻ steht, wobei X⁻ für das Anion einer Säure, bevorzugt für Chlorid oder Acetat steht,
in Gegenwart des Alkalimetallalkoholats der Formel (I),
wobei das Alkalimetallalkoholat der Formel (I) in Schritt a) durch Umsetzung von zumindest einem Alkalihydroxid der Formel (IV),
M-OH (IV),
in der M die in Formel (I) angegebene Bedeutung hat,
mit einem Alkohol der Formel (V),
R¹-OH (V)
in der R¹ die in Formel (I) genannte Bedeutung hat, entweder in Reinsubstanz oder in Form einer Mischung,
unter Abdestillieren von Wasser und Alkohol der Formel (V), bis der Destillationsrückstand einen Gehalt an Alkalihydroxid der Formel (IV) von maximal 1000 mg/kg, bevorzugt von maximal 300 mg/kg, bezogen auf das Gesamtgewicht der Mischung, aufweist,
bereitgestellt wird.

2. Verfahren nach Anspruch 1, wobei die Umsetzung von Malonsäureester der Formel (II) und Ameisensäurederivat der Formel (III) in Gegenwart des Alkalimetallalkoholats der Formel (I) in Schritt b) bei einer Temperatur von 50 bis 110°C, bevorzugt von 60 bis 80°C, erfolgt.

3. Verfahren nach einem der Ansprüche 1 oder 2, wobei in Schritt b) das Alkalimetallalkoholat der Formel (I) in Form einer Suspension, einer Schmelze oder als Lösung, vorzugsweise mit dem korrespondierenden Alkohol der Formel (V) als Lösungsmittel, vorliegt.

4. Verfahren nach einem der Ansprüche 1 bis 3, wobei in Schritt b) die Umsetzung von Malonsäureester der Formel (II) und Ameisensäurederivat der Formel (III) in Gegenwart des Alkalimetallalkoholats der Formel (I) derart erfolgt, dass Malonsäureester der Formel (II) und Ameisensäurederivat der Formel (III) zu dem Alkalimetallalkoholat der Formel (I) zugegeben werden, wobei während eines beliebigen Zeitpunktes der Zugabe das molare Verhältnis der Summe des bis zu diesem Zeitpunkt zugegebenen Ameisensäurederivats der Formel (III) zu der Summe des bis zu diesem Zeitpunkt zugegebenen Malonsäureesters der Formel (II) mindestens 2,05, bevorzugt mindestens 2,5 bis 3,5, beträgt.

5. Verfahren nach einem der Ansprüche 1 bis 4, wobei in einem Schritt
c) die bei Schritt b) resultierenden Mischung bei einer Temperatur von 50 bis 110°C, bevorzugt von 60 bis 80°C, umgesetzt wird.

6. Verfahren nach einem der Ansprüche 1 bis 5, umfassend den Schritt
d) Inkontaktbringen von anorganischer Säure und Wasser mit der Reaktionsmischung aus Schritt c) in einer Menge, die ausreicht, um die Mischung auf einen pH-Wert von 2 bis 5, bevorzugt von pH 3 bis 4, zu bringen.

7. Verfahren nach einem der Ansprüche 1 bis 6 umfassend den Schritt
e) Trennung des aus Schritt d) erhaltenen Reaktionsgemisches, wobei 4,6-Dihydroxypyrimidin als Feststoff und ein zweiphasiges Filtrat erhalten wird, wobei eine Phase zumindest überwiegend den Alkohol der Formel (V) enthält.

8. Verfahren nach einem der Ansprüche 1-7, in dem das gemäß Schritt e) erhaltenen zweiphasige Filtrat aufgetrennt wird und die dabei erhaltene zumindest überwiegend den Alkohol der Formel (V) enthaltende Phase destilliert und wieder zur Herstellung des Alkalimetallalkoholats der Formel (I) gemäß Anspruch 1 eingesetzt wird.

9. Verfahren nach einem der Ansprüche 1 bis 8, wobei in Schritt b) die Umsetzung durch die Zugabe von Malonsäureester der Formel (II) und Ameisensäurederivat der Formel (III) zu dem Alkalimetallalkoholat der Formel (I) diskontinuierlich oder kontinuierlich erfolgt.

10. Verfahren nach einem der Ansprüche 1 bis 9, wobei in dem in Schritt a) hergestellten Alkalimetallalkoholat der Formel (I) der Rest R¹ für n-Butyl und der Rest M für Natrium oder Kalium steht.

11. Verfahren nach einem der Ansprüche 1 bis 10, in dem das Ameisensäurederivat der Formel (III) ein Formamidiniumsalz ausgewählt aus Formamidiniumacetat oder Formamidiniumhydrochlorid ist.

12. Verfahren nach einem der Ansprüche 1 bis 11, wobei das Inkontaktbringen von anorganischer Säure und Wasser zu der Mischung aus Schritt c) in Schritt d) diskontinuierlich oder kontinuierlich erfolgt.

13. Verfahren nach einem der Ansprüche 1 bis 12, **dadurch gekennzeichnet, dass** als Malonsäureester der Formel (II) der Dimethyl-, der Diethyl- oder der Di-n-butylester von Malonsäure verwendet wird.

14. Verwendung der Alkalimetallalkoholate der Formel (I) zur Herstellung von 4,6-Dihydroxypyrimidin.

## Claims

1. Method for preparing 4,6-dihydroxypyrimidine, comprising at least the steps of
a) providing an alkali metal alkoxide of the formula (I),
R¹-OM (I)
in which R¹ is n-butyl, isobutyl and sec-butyl and M is sodium and potassium,
b) reacting malonic ester of the formula (II), 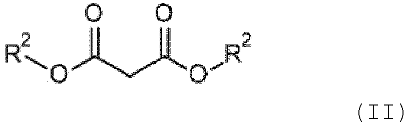
in which R² is C₁ to C₄ alkyl,
with a formic acid derivative of the formula (III), 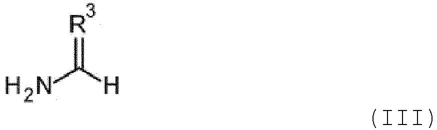
in which the radical R³ is O, HN or NH⁺X⁻, where X⁻ is the anion of an acid, preferably chloride or acetate,
in the presence of the alkali metal alkoxide of the formula (I),
wherein the alkali metal alkoxide of the formula (I) in step a) is provided by reacting at least one alkali metal hydroxide of the formula (IV),
M-OH (IV),
in which M is as defined in formula (I),
with an alcohol of the formula (V),
R¹-OH (V)
in which R¹ is as defined in formula (I), either in the form of the pure substance or as a mixture,
water and alcohol of the formula (V) being distilled off until the distillation residue has a content of alkali metal hydroxide of the formula (IV) of not more than 1000 mg/kg, preferably of not more than 300 mg/kg, based on the total weight of the mixture.

2. Method according to Claim 1, wherein the reaction of malonic ester of the formula (II) and formic acid derivative of the formula (III) in the presence of the alkali metal alkoxide of the formula (I) in step b) takes place at a temperature of 50 to 110°C, preferably of 60 to 80°C.

3. Method according to either of Claims 1 or 2, wherein in step b) the alkali metal alkoxide of the formula (I) is present in the form of a suspension, a melt or a solution, preferably with the corresponding alcohol of the formula (V) as solvent.

4. Method according to any of Claims 1 to 3, wherein in step b) the reaction of malonic ester of the formula (II) and formic acid derivative of the formula (III) in the presence of the alkali metal alkoxide of the formula (I) takes place such that malonic ester of the formula (II) and formic acid derivative of the formula (III) are added to the alkali metal alkoxide of the formula (I), wherein, during any point in the addition, the molar ratio of the total amount of formic acid derivative of the formula (III) added up to that point to the total amount of malonic ester of the formula (II) added up to that point is at least 2.05, preferably at least 2.5 to 3.5.

5. Method according to any of Claims 1 to 4, wherein in a step
c) the mixture resulting in step b) is reacted at a temperature of 50 to 110°C, preferably of 60 to 80°C.

6. Method according to any of Claims 1 to 5, comprising the step of
d) contacting of inorganic acid and water with the reaction mixture from step c) in an amount that is sufficient to bring the mixture to a pH of 2 to 5, preferably of pH 3 to 4.

7. Method according to any of Claims 1 to 6, comprising the step of
e) separating the reaction mixture obtained from step d), affording 4,6-dihydroxypyrimidine in solid form and a two-phase filtrate in which one phase at least mostly comprises the alcohol of the formula (V).

8. Method according to any of Claims 1-7 in which the two-phase filtrate obtained in step e) is separated and the resulting phase at least mostly comprising the alcohol of the formula (V) is distilled and reused for the preparation of the alkali metal alkoxide of the formula (I) according to Claim 1.

9. Method according to any of Claims 1 to 8, wherein in step b) the reaction through the addition of malonic ester of the formula (II) and formic acid derivative of the formula (III) to the alkali metal alkoxide of the formula (I) takes place discontinuously or continuously.

10. Method according to any of Claims 1 to 9, wherein, in the alkali metal alkoxide of the formula (I) produced in step a), the radical R¹ is n-butyl and the radical M is sodium or potassium.

11. Method according to any of Claims 1 to 10, in which the formic acid derivative of the formula (III) is a formamidinium salt selected from formamidinium acetate or formamidinium hydrochloride.

12. Method according to any of Claims 1 to 11, wherein the contacting in step d) of inorganic acid and water with the mixture from step c) takes place discontinuously or continuously.

13. Method according to any of Claims 1 to 12, **characterized in that** the employed malonic ester of the formula (II) is dimethyl, diethyl or di-n-butyl malonate.

14. Use of alkali metal alkoxides of the formula (I) in the preparation of 4,6-dihydroxypyrimidine.

## Revendications

1. Procédé de préparation de 4,6-dihydroxypyrimidine, comprenant au moins les étapes suivantes :
a) fourniture d'un alcoolate d'un métal alcalin de Formule (I),
R¹-OM (I)
dans laquelle R¹ représente un n-butyle, un isobutyle et un sec-butyle et M représente le sodium et le potassium,
b) réaction de l'ester de l'acide malonique de Formule (II), 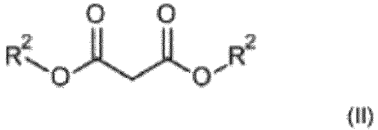 dans laquelle R² représente un alkyle en C₁ à C₄, avec un dérivé de l'acide formique de Formule (III), 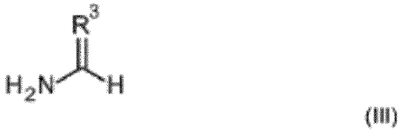
dans laquelle le radical R³ représente O, HN ou NH⁺X⁻, X⁻ étant l'anion d'un acide, de préférence un chlorure ou un acétate,
en présence de l'alcoolate d'un métal alcalin de Formule (I),
l'alcoolate d'un métal alcalin de Formule (I) étant préparé dans l'étape a) par réaction d'au moins un hydroxyde d'un métal alcalin de Formule (IV),
M-OH (IV),
dans laquelle M a les significations données dans la Formule (I),
avec un alcool de Formule (V),
R¹-OH (V)
dans laquelle R¹ a les significations données dans la Formule (I), sous forme de substance pure ou d'un mélange,
par élimination par distillation de l'eau et de l'alcool de Formule (V) jusqu'à ce que le résidu de distillation présente une teneur en l'hydroxyde d'un métal alcalin de Formule (IV) au maximum de 1 000 mg/kg, de préférence au maximum de 300 mg/kg, par rapport au poids total du mélange.

2. Procédé selon la revendication 1, dans lequel la réaction de l'ester de l'acide malonique de Formule (II) et du dérivé de l'acide formique de Formule (III) a lieu en présence de l'alcoolate d'un métal alcalin de Formule (I) dans l'étape b) à une température de 50 à 110 °C, de préférence de 60 à 80 °C.

3. Procédé selon l'une des revendications 1 ou 2, dans lequel dans l'étape b) l'alcoolate d'un métal alcalin de Formule (I) se présente sous forme d'une suspension, d'une masse fondue ou d'une solution, de préférence avec l'alcool correspondant de Formule (V) servant de solvant.

4. Procédé selon l'une des revendications 1 à 3, dans lequel dans l'étape b) la réaction de l'ester de l'acide malonique de Formule (II) et du dérivé de l'acide formique de Formule (III) a lieu en présence de l'alcoolate d'un métal alcalin de Formule (I) par le fait que l'on ajoute l'ester de l'acide malonique de Formule (II) et le dérivé de l'acide formique de Formule (III) à l'alcoolate d'un métal alcalin de Formule (I), procédé dans lequel, pendant un instant quelconque de l'addition, le rapport en moles de la somme du dérivé de l'acide formique de Formule (III) ajouté à cet instant-là à la somme de l'ester de l'acide malonique de Formule (II) ajouté à cet instant-là est d'au moins 2,05, de préférence d'au moins 2,5 à 3,5.

5. Procédé selon l'une des revendications 1 à 4, dans lequel on fait réagir dans une étape c) le mélange obtenu dans l'étape b), à une température de 50 à 110 °C, de préférence de 60 à 80 °C.

6. Procédé selon l'une des revendications 1 à 5, comprenant l'étape
d) mise en contact d'un acide inorganique et d'eau avec le mélange réactionnel de l'étape c) en une quantité suffisante pour porter le mélange à un pH de 2 à 5, de préférence à un pH de 3 à 4.

7. Procédé selon l'une des revendications 1 à 6, comprenant l'étape
e) séparation du mélange réactionnel obtenu dans l'étape d), avec obtention de 4,6-dihydroxypyrimidine sous forme d'un solide et d'un filtrat biphasique, une phase contenant au moins essentiellement l'alcool de Formule (V).

8. Procédé selon l'une des revendications 1 à 7, dans lequel on sépare le filtrat biphasique obtenu dans l'étape e), et on distille la phase obtenue à cette occasion, contenant au moins essentiellement l'alcool de Formule (V), et on la réutilise pour préparer l'alcoolate d'un métal alcalin de Formule (I) selon la revendication 1.

9. Procédé selon l'une des revendications 1 à 8, dans lequel dans l'étape b), la réaction est mise en œuvre par addition continue ou discontinue de l'ester de l'acide malonique de Formule (II) et du dérivé de l'acide formique de Formule (III) à l'alcoolate d'un métal alcalin de Formule (I).

10. Procédé selon l'une des revendications 1 à 9, dans lequel, dans l'alcoolate d'un métal alcalin de Formule (I) préparé dans l'étape a), le radical R¹ représente un n-butyle et le radical M représente un sodium ou un potassium.

11. Procédé selon l'une ou plusieurs des revendications 1 à 10, dans lequel le dérivé de l'acide formique de Formule (III) est un sel de formamidinium choisi parmi l'acétate de formamidinium ou le chlorhydrate de formamidinium.

12. Procédé selon l'une des revendications 1 à 11, dans lequel la mise en contact de l'acide inorganique et de l'eau avec le mélange de l'étape c) est mise en œuvre d'une manière continue ou discontinue dans l'étape d).

13. Procédé selon l'une des revendications 1 à 12, **caractérisé en ce qu'**on utilise en tant qu'ester de l'acide malonique de Formule (II) l'ester diméthylique, diéthylique ou di-n-butylique de l'acide malonique.

14. Utilisation des alcoolates d'un métal alcalin de Formule (I) pour la préparation de 4,6-dihydroxypyrimidine.
